# NEW EUROPEAN PATENT SPECIFICATION

(11) **EP 0 524 211 B2**
(45) Date of publication and mention of the opposition decision: **11.08.1999**
(45) Mention of the grant of the patent: 08.03.1995
(21) Application number: 91907023.5
(22) Date of filing: 02.04.1991
(51) Int. Cl.: A61K 9/46, A61K 31/43

(54) **PHARMACEUTICAL FORMULATION**
ARZNEIMITTEL
FORMULATION PHARMACEUTIQUE

(30) Priority: 07.04.1990 GB 9007945
(43) Date of publication of application: 27.01.1993
(73) Proprietor: BEECHAM GROUP PLC, Brentford, Middlesex TW8 9EP (GB)
(72) Inventor: MERRIFIELD, David Roy, Worthing, West Sussex BN14 8QH (GB); CARTER, Paul Laurence, West Sussex BN14 8QH (GB); DOUGHTY, David George, SmithKline Beecham Pharm., Epsom, Surrey KT18 5XQ (GB)
(74) Representative: Walker, Ralph Francis, Dr.
(86) International application number: GB9100516
(87) International publication number: WO9115197

(56) References cited:
- EP-A- 0 080 862
- EP-A- 0 207 041
- EP-A- 0 281 200
- EP-A- 0 293 975
- EP-A- 0 351 353
- EP-A- 0 396 335
- DE-A- 1 938 709
- DE-A- 2 020 893
- DE-A- 2 517 316
- DE-A- 2 750 207
- DE-A- 2 843 318
- GB-A- 1 221 038
- GB-A- 1 300 998
- NL-C- 146 377
- US-A- 3 105 792
- US-A- 3 495 001
- US-A- 3 969 524
- US-A- 4 888 177
- RO-79019 and CA 99 (1983) n° 16, 128342z
- L. Lachman et al. The Theory and practice of industrial pharmacy, 1986, 3rd ed. pages 334, 335, 344 and 345
- H.A. Lieberman et al. Pharmaceutical dosage-forms : tablets, vol. 1, 1980, pages 226-229, 232, 241-242
- Package of FlemoxinR effervescent tablets and package insert
- J. Pharm. Sc. vol. 67 (1978) n° 8, pages 1059-1066
- Martindale The Extra Pharmacopoeia, 28th ed. (1982) and 30rd ed. (1993), monographs on amoxycillin and ampicillin
- K. Florey : Analytical profiles of drug substances, vol. 7 (1978), pages 22 and 34
- British Pharmacopoeia, vol. II (1988), page 893

## Description

The present invention relates to pharmaceutical compositions for oral administration in the treatment of bacterial infections.

In some clinical situations, to improve patient compliance, it is desirable to administer medicaments orally in liquid form as suspensions or solutions.

EP-A-0080862 (Beecham) discloses water-dispersible compositions of amoxycillin trihydrate, in which the amoxycillin trihydrate and other ingredients are formulated with a non-hygroscopic water-soluble binder.

Solutions are favoured over suspensions for oral administration, since drugs in solution are more rapidly absorbed. Solutions are also often more acceptable to patients, in terms of palatability. It has been proposed to prepare dry effervescent formulations of medicaments in which, on addition to water, a medicament is dispersed in the water by the effervescing action and dissolves either as a result of the agitation or by interaction with components of the formulation. For example, GB-A-1287475 (Aspro-Nicholas) describes an effervescent formulation of aspirin. In order to obtain effective contact of the aspirin with the solubilising compounds during effervescence, the aspirin particles are pre-coated with a special readily wettable coating.

Effervescent formulations of antibiotics are disclosed in GB-A-1300998 (Biochemie). In this disclosure it is considered essential that the antibiotic is in the form of a water-soluble salt in the dry formulation. For amoxycillin this would be disadvantageous because the water-soluble sodium salt is very hygroscopic and unstable when it has absorbed water.

A dispersible tablet formulation containing amoxycillin is disclosed in EP-0281200-A1 (Gist-Brocades). This formulation does not result in a clear solution of dissolved amoxycillin, but a suspension.

We have now discovered that amoxycillin that is not in salt form can be provided as an effervescent formulation in which it is solubilised on contact with water, and in particular that will produce a clear solution for oral administration.

According to the present invention there is provided a pharmaceutical formulation for oral administration comprising an amoxycillin hydrate and an effervescent couple which comprises an acid component and an alkaline component, which generates carbon dioxide on contact with water, in which the alkaline component of the couple is present in excess of the stoichiometric equivalent of the acid component in a sufficient amount to both neutralise the acid component and to solubilise the amoxycillin completely.

The amoxycillin hydrate is preferably amoxycillin trihydrate and may be provided in conjunction with a β-lactamase inhibitor, such as clavulanic acid or a salt thereof preferably potassium clavulanate. A suitable ratio range of amoxycillin: clavulanic acid or clavulanate salt equivalent is 12:1 to 1:1, preferably 7:1 to 1:1, 4:1 to 1:1 or 2:1 to 1:1, by weight. A suitable proportion of amoxycillin in the formulation is 10-30% by weight, e.g. 10-25%.

The effervescent couple is preferably based on citric acid and sodium bicarbonate or sodium glycine carbonate, but other solid acid/carbonate couples may be used, for example tartaric or malic acid and sodium carbonate or potassium bicarbonate or mixtures of these acid and alkaline components. The effervescent couple is provided in a sufficient amount to rapidly disperse and assist dissolution of the components of the formulation. The corresponding potassium salts of the alkaline component may be used together with the sodium salts (or as a substitute) to avoid excessive levels of sodium ions. This may be necessary when high doses of amoxycillin are included in the composition.

The alkaline component should be present in sufficient amount to both neutralize the acid component and to solubilise the amoxycillin by formation of soluble e.g. sodium/potassium, salts. The aim is that the resulting aqueous solution should have a pH of not less than 8 to achieve solubilization of amoxycillin trihydrate. Typically the composition may contain 50-75% of an alkaline component such as sodium or potassium hydrogen carbonate or glycine carbonate, by weight. A suitable mixed alkaline component is a 3-1.5:1, for example 2.5-2:1 by weight mixture of sodium glycine carbonate: potassium bicarbonate.

Typically the composition may contain 2-25%, e.g. 5-20%, e.g. 5-17.5%, by weight of an acid component such as citric acid.

The amounts of effervescent couple and excess alkaline component required to achieve rapid and complete solubilisation of a particular amoxycillin dosage can be determined by simple experiments. For doses of amoxycillin of 1g or more, suitable ranges of molar ratios of sodium glycine carbonate: amoxycillin: potassium bicarbonate: citric acid in the formulation are 4-10: 1-3: 5-10: 1, for example 5-8: 1.5-2.5: 6.5-7.5: 1. Citric acid is tribasic, and suitable molar ratios of other acids may be calculated accordingly. The suitable molar ratio expressed above corresponds to a weight ratio sodium glycine carbonate 4.8-12: amoxycillin 1.9-5.7: potassium bicarbonate 2-6-5.1: citric acid 1, with a preferred weight ratio of sodium glycine carbonate: amoxycillin of at least 1.66.

Suitable ranges of molar ratios of sodium glycine carbonate: amoxycillin: citric acid are 1.5-4.5: 0.2-1: 1, provided that the alkaline component is present in excess of the stoichiometric equivalent of the acid component in an amount sufficient to both neutralise the acid component and to solubilise the amoxycillin completely. The suitable molar ratio expressed above corresponds to a weight ratio sodium glycine carbonate: amoxycillin: citric acid of 1.7-5.5: 0.4-1.9: 1.

For lower doses of amoxycillin, for example 500mg, 250mg and 125mg the levels of sodium ions is not excessive and the inclusion of potassium bicarbonate is not necessary.

Conventional excipients, such as colourings, fillers, diluents, sweeteners and flavourings may be added to the formulations, typically in an amount up to around 10% by weight, e.g. 1-7.5%. A suitable sweetener is aspartame.

The formulations are typically in the form of free flowing powders or granules, or tablets.

Soluble tablets may contain conventional water-soluble lubricants such as sodium lauryl sulphate or sodium benzoate, typically up to around 7.5% or less. Alternatively tablets may be made using external lubrication on liquid-lubricated presses, or on double-sided presses where solid lubricant placebo compacts containing, for example, magnesium stearate are made on one side, continuously pre-lubricating the dies. The manufacturing method may be entirely conventional, e.g. formation of a granulate intermediate containing some or all of the milled components, followed by optionally blending with the other components and then pressing into tablets.

Soluble tablets are preferably conventionally packaged in protective containers such as screw cap bottles, aluminium foil sachets, plastics or metal tubes, or aluminium blister packs. Soluble powders or granules are preferably conventionally packaged in individual aluminium foil sachets, each containing a unit dose of the antibiotic. It may be appropriate to incorporate a desiccant in the packaging.

The amount of amoxycillin in a unit dose will depend on the infection to be treated and the assay of the amoxycillin. The unit-dose will be repeated according to the usual regime for amoxycillin treatments. Typically a unit dose may contain 3000, 875 or 125 mg of amoxycillin per tablet or sachet, or an intermediate dose.

The invention also provides a formulation as defined above for use in the treatment of bacterial infections in humans or animals.

The invention also provides a process for the preparation of a pharmaceutical formulation for oral administration which comprises admixing an amoxycillin hydrate and an effervescent couple, the couple comprising an acid component and an alkaline component which generates carbon dioxide on contact with water, the alkaline component of the couple being present in excess of the stoichiometric equivalent of the acid component in a sufficient amount to both neutralise the acid component and to solubilise the amoxycillin completely.

The invention also provides a use, of an admixture of an amoxycillin hydrate and an effervescent couple, the couple comprising an acid component and an alkaline component which generates carbon dioxide on contact with water, the alkaline component of the couple being present in excess of the stoichiometric equivalent of the acid component in a sufficient amount to both neutralise the acid component and to solubilise the amoxycillin completely, in the manufacture of a medicament for oral administration, for the treatment of bacterial infections.

The invention is illustrated by the following Examples.

### Example 1

### 3g Dose Soluble Sachet

| Ingredients | g/dose | % w/w |
|---|---|---|
| Amoxycillin trihydrate (as free acid) | 3.000 | 22.5 |
| Potassium bicarbonate | 2.800 | 21.04 |
| Sodium glycine carbonate | 6.212 | 46.7 |
| Citric acid | 0.800 | 6.01 |
| Aspartame | 0.150 | 1.13 |
| Sodium saccharin | 0.040 | 0.30 |
| Lemon juice flavour | 0.220 | 1.65 |
| Cinnamon flavour | 0.085 | 0.64 |

Reconstitution: Add the contents of each sachet to 200mls of water and stir gently.

### Manufacturing Process

The amoxycillin trihydrate was passed through an Apex 114 mill fitted with a 0.027 inch (0.686 mm) aperture screen using hammers forward at 4590 rpm.

The potassium bicarbonate, sodium glycine carbonate, aspartame, dried saccharin sodium and citric acid were passed through a 30 mesh screen and placed in a blender with the milled amoxycillin trihydrate. The mix was blended for 20 minutes at slow speed. The blend was then passed through a roller compactor, and the compact passed through an Apex 114 mill fitted with a 0.063 inch (1.6 mm) aperture screen, using knives forward at 2880 rpm, into a blender. The flavours were screened through a 20 mesh screen into the blender, and the mix blended for 15 minutes at slow speed. The final mixture was filled into sachets at a weight calculated to deliver the required dose of amoxycillin.

### Example 2

### 1g Dose Soluble Sachets

| Ingredients | mg/sachet | (% w/w) |
|---|---|---|
| Amoxycillin Trihydrate equivalent to Amoxycillin free acid | 875.0 | 19.06 |
| Potassium Clavulanate equivalent to Clavulanic acid | 125.0 | 2.72 |
| Potassium bicarbonate | 930.0 | 20.26 |
| Citric acid (anhydrous) | 270.0 | 5.88 |
| Aspartame | 40.0 | 0.87 |
| Sodium saccharin | 10.4 | 0.23 |
| Lemon dry flavour | 73.0 | 1.59 |
| Cinnamon flavour | 28.0 | 0.61 |
| Sodium glycine carbonate | 2238.6 | 48.77 |

### Manufacturing Process

The amoxycillin trihydrate was passed through an Apex 114 mill fitted with a 0.027 inch (0.686 mm) screen using hammers forward at 4,600 rpm. All other ingredients were passed though a 30 mesh screen. The reduced amoxycillin trihydrate and other ingredients were blended in a suitably sized Y-cone blender for 20 minutes. The resultant mixture was compacted on a roller compactor, and the compact was reduced to granules and classified.

### Example 3

### 3.25g Dose Soluble Sachet

| Ingredients | mg/dose | % (w/w) |
|---|---|---|
| Amoxycillin trihydrate | 3000 (as free acid) | 25.0 |
| Potassium clavulanate | 250 (as free acid) | 2.08 |
| Sodium glycine carbonate | 4968 | 41.39 |
| Potassium bicarbonate | 2504 | 20.86 |
| Citric acid anhydrous | 640 | 5.33 |
| Aspartame | 150 | 1.25 |
| Sodium saccharin | 40 | 0.33 |
| Golden syrup flavour | 150 | 1.25 |
| Banana flavour | 300 | 2.5 |

### Example 4

### 156.3mg Dose Soluble Tablet

| Ingredients | mg/tablet | % (w/w) |
|---|---|---|
| Amoxycillin trihydrate | 125.00 (as free acid) | 10.43 |
| Potassium clavulanate | 31.25 (as free acid) | 2.61 |
| Sodium glycine carbonate | 625.00 | 52 |
| Citric acid anhydrous | 200.00 | 16.69 |
| Sodium benzoate | 66.90 | 5.58 |
| Aspartame | 37.50 | 3.13 |
| Golden syrup flavour | 37.50 | 3.13 |
| Banana flavour | 75.00 | 6.26 |

This tablet is compressed on ⁹/16 inch (14.288 mm) bevel-flat punches.

### Example 5

### 125mg Dose Soluble Tablets

| Ingredients | mg/tablet | % (w/w) |
|---|---|---|
| Amoxycillin trihydrate | 125.0 (as free acid) | 18.89 |
| Sodium glycine carbonate | 406.3 | 61.39 |
| Citric acid anhydrous | 93.8 | 14.17 |
| Aspartame | 12.1 | 1.83 |
| Lemon juice flavour | 17.8 | 2.69 |
| Cinnamon flavour | 6.8 | 1.03 |

### Manufacturing Process

The amoxycillin trihydrate was passed through an Apex 114 mill fitted with a 0.027 inch (0.686 mm) screen, hammers forward, at 7200 rpm into a blender. The citric acid was passed through an Apex 114 mill fitted with a 0.040 inch (1 mm) screen, hammers forward, at 7200 rpm into the blender. The other ingredients except for the flavours were passed though a 30 mesh screen into the blender. The mix was blended for 20 minutes, and the blend slugged on one side of a Manesty BB4 double-sided press fitted with ½ inch (12.5 mm) round bevelled flat tooling. A lubricating mix consisting of 3% magnesium stearate in lactose was compressed on the other side of the machine. The slugs were milled on an Apex 114 mill fitted with a 0.063 (1.6 mm) inch screen, knives forward at 2900 rpm. The flavours were passed through a 30 mesh screen and blended with the reduced slugs for 20 minutes. The blend was compressed on the double-sided press fitted with the same tooling as used to prepare the slugs, and lubricated in the same manner.

### Example 6

### 250mg Dose Soluble Tablets

250mg tablets were prepared by exactly doubling the quantities described in Example 5, and using an identical process except for replacing the ½ inch (12.5 mm) punches by 5/8 inch (15.875 mm) punches.

In the formulations of Examples 1-6 above the relative proportions of components are preferably maintained within ± 10% of the stated quantities.

## Claims

1. A pharmaceutical formulation for oral administration comprising an amoxycillin hydrate and an effervescent couple, the couple comprising an acid component and an alkaline component, which generates carbon dioxide on contact with water, in which the alkaline component of the couple is present in excess of the stoichiometric equivalent of the acid component in a sufficient amount to both neutralise the acid component and to solubilise the amoxycillin completely.

2. A pharmaceutical formulation according to claim 1 in which the amoxycillin hydrate is amoxycillin trihydrate and/or is in conjunction with a β-lactamase inhibitor.

3. A pharmaceutical formulation according to claim 2 in which a β-lactamase inhibitor is present and is clavulanic acid or a salt thereof, in a weight ratio of 12:1 to 1:1 amoxycillin hydrate : inhibitor.

4. A pharmaceutical formulation according to any one of the preceding claims in which the proportion of amoxycillin hydrate is 10 - 30% by weight.

5. A pharmaceutical formulation according to any one of the preceding claims in which the effervescent couple is selected from citric acid, tartaric acid or malic acid or mixtures thereof as acid component, and sodium bicarbonate, sodium glycine carbonate or sodium carbonate, or the corresponding potassium salts, or mixtures thereof as the alkaline component.

6. A pharmaceutical formulation according to any one of the preceding claims which when made up into aqueous solution has a pH of not less than 8.

7. A pharmaceutical formulation according to any one of the preceding claims which contains 50 - 75 weight % of alkaline component.

8. A pharmaceutical formulation according to any one of the preceding claims in which the alkaline component is a 3 - 1.5 : 1 by weight mixture of sodium glycine carbonate or sodium bicarbonate : potassium bicarbonate.

9. A pharmaceutical formulation according to any one of the preceding claims which contains 2 - 25 weight % of the acid component.

10. A pharmaceutical formulation according to any one of the preceding claims containing a molar ratio of sodium glycine carbonate : amoxycillin : potassium bicarbonate : citric acid in the range 4-10 : 1- 3 : 5-10 : 1.

11. A pharmaceutical formulation according to claim 1 being a free flowing powder or granule formulation and having a composition within ± 10% of :
| Ingredient | % w/w |
|---|---|
| Amoxycillin trihydrate as free acid | 22.5 |
| Potassium bicarbonate | 21.04 |
| Sodium glycine carbonate | 46.7 |
| Citric acid | 6.01 |
| Aspartame | 1.13 |
| Sodium saccharin | 0.30 |
| Lemon juice flavour | 1.65 |
| Cinnamon flavour | 0.64 |

12. A pharmaceutical formulation according to claim 1 being a free flowing powder or granule formulation having a composition within ± 10% of :
| Ingredients | % w/w |
|---|---|
| Amoxycillin Trihydrate equivalent to Amoxycillin free acid | 19.06 |
| Potassium Clavulanate equivalent to Clavulanic acid | 2.72 |
| Potassium bicarbonate | 20.26 |
| Citric acid (anhydrous) | 5.88 |
| Aspartame | 0.87 |
| Sodium saccharin | 0.23 |
| Lemon dry flavour | 1.59 |
| Cinnamon flavour | 0.61 |
| Sodium glycine carbonate | 48.77 |

13. A pharmaceutical formulation according to claim 1 being a free flowing powder or granule formulation having a composition within ± 10% of :
| Ingredients | % w/w |
|---|---|
| Amoxycillin trihydrate as free acid | 25.0 |
| Potassium clavulanate as free acid | 2.08 |
| Sodium glycine carbonate | 41.39 |
| Potassium bicarbonate | 20.86 |
| Citric acid anhydrous | 5.33 |
| Aspartame | 1.25 |
| Sodium saccharin | 0.33 |
| Golden syrup flavour | 1.25 |
| Banana flavour | 2.5 |

14. A pharmaceutical formulation according to claim 1 being a tablet formulation having a composition within ± 10% of :
| Ingredients | % w/w |
|---|---|
| Amoxycillin trihydrate as free acid | 10.43 |
| Potassium clavulanate as free acid | 2.61 |
| Sodium glycine carbonate | 52.16 |
| Citric acid anhydrous | 16.69 |
| Sodium benzoate | 5.58 |
| Aspartame | 3.13 |
| Golden syrup flavour | 3.13 |
| Banana flavour | 6.26 |

15. A pharmaceutical formulation according to claim 1 being a tablet formulation having a composition within ± 10% of :
| Ingredients | % w/w |
|---|---|
| Amoxycillin trihydrate as free acid | 18.89 |
| Sodium glycine carbonate | 61.39 |
| Citric acid anhydrous | 14.17 |
| Aspartame | 1.83 |
| Lemon juice flavour | 2.69 |
| Cinnamon flavour | 1.03 |

16. A pharmaceutical formulation according to any one of the preceding claims, containing a unit dose of amoxycillin between 3000 and 125 mg inclusive.

17. A pharmaceutical formulation according to any one of the preceding claims, for use in the treatment of bacterial infections in humans or animals.

18. A process for the preparation of a pharmaceutical formulation for oral administration which comprises admixing an amoxycillin hydrate and an effervescent couple, the couple comprising an acid component and an alkaline component which generates carbon dioxide on contact with water, the alkaline component of the couple being in excess of the stoichiometric equivalent of the acid component, in a sufficient amount to both neutralise the acid component and to solubilise the amoxycillin completely.

19. The use of an admixture of an amoxycillin hydrate and an effervescent couple, the couple comprising an acid component and an alkaline component which generates carbon dioxide on contact with water, the alkaline component of the couple being present in excess of the stoichiometric equivalent of the acid component in a sufficient amount to both neutralise the acid component and to solubilise the amoxycillin completely, in the manufacture of a medicament for oral administration for the treatment of bacterial infections.

## Patentansprüche

1. Pharmazeutische Formulierung zur oralen Verabreichung, umfassend ein Amoxycillinhydrat und ein Brausepaar, wobei das Paar eine saure Komponente und eine alkalische Komponente umfaßt und bei Kontakt mit Wasser Kohlendioxid freisetzt, wobei die alkalische Komponente des Paares im Überschuß zum stöchiometrischen Äquivalent der sauren Komponente in solcher Menge vorliegt, die ausreicht, um sowohl die saure Komponente zu neutralisieren als auch das Amoxycillin vollständig aufzulösen.

2. Pharmazeutische Formulierung gemäß Anspruch 1, wobei das Amoxycillinhydrat Amoxycillintrihydrat ist und/oder in Verbindung mit einem β-Lactamase-Inhibitor vorliegt.

3. Pharmazeutische Formulierung gemäß Anspruch 2, wobei ein β-Lactamase-Inhibitor vorliegt, nämlich Clavulansäure oder deren Salz in einem Gewichtsverhältnis von 12:1 bis 1:1 Amoxycillinhydrat:Inhibitor.

4. Pharmazeutische Formulierung gemäß einem der vorstehenden Ansprüche, wobei der Amoxycillinhydratanteil 10-30 Gew.-% beträgt.

5. Pharmazeutische Formulierung gemäß einem der vorstehenden Ansprüche, wobei das aufschäumende Paar aus Citronensäure, Weinsäure oder Äpfelsäure oder Gemischen daraus als saure Komponente, und Natriumbicarbonat, Natriumglycincarbonat oder Natriumcarbonat oder den entsprechenden Kaliumsalzen oder Gemischen daraus als alkalische Komponente ausgewählt ist.

6. Pharmazeutische Formulierung gemäß einem der vorstehenden Ansprüche, die bei Zubereitung in wäßriger Losung einen pH-Wert von nicht weniger als 8 hat.

7. Pharmazeutische Formulierung gemäß einem der vorstehenden Ansprüche, die 50-75 Gew.-% alkalische Komponente enthält.

8. Pharmazeutische Formulierung gemäß einem der vorstehenden Ansprüche, wobei die alkalische Komponente ein Gemisch aus Natriumglycincarbonat oder Natriumbicarbonat:Kaliumbicarbonat im Verhältnis von 3-1,5:1 auf das Gewicht bezogen ist.

9. Pharmazeutische Formulierung gemäß einem der vorstehenden Ansprüche, die 2-25 Gew.-% der sauren Komponente enthält.

10. Pharmazeutische Formulierung gemäß einem der vorstehenden Ansprüche, mit einem Molverhältnis von Natriumglycincarbonat:Amoxycillin:Kaliumbicarbonat:Citronensäure im Bereich von 4-10:1-3:5-10:1.

11. Pharmazeutische Formulierung gemäß Anspruch 1, die ein freifließendes Pulver oder eine körnige Formulierung ist mit folgender Zusammensetzung innerhalb von ± 10%:
| Inhaltsstoff | Gew.-% |
|---|---|
| Amoxycillintrihydrat als freie Säure | 22,5 |
| Kaliumbicarbonat | 21,04 |
| Natriumglycincarbonat | 46,7 |
| Citronensäure | 6,01 |
| Aspartam | 1,13 |
| Natriumsaccharin | 0,30 |
| Zitronensaftaroma | 1,65 |
| Zimtaroma | 0,64 |

12. Pharmazeutische Formulierung gemäß Anspruch 1, die ein freifließendes Pulver oder eine körnige Formulierung ist mit folgender Zusammensetzung innerhalb von ±10%:
| Inhaltsstoff | Gew.-% |
|---|---|
| Amoxycillintrihydrat, äquivalent zu Amoxycillin als freier Säure | 19,06 |
| Kaliumclavulanat, äquivalent zu Clavulansäure | 2,72 |
| Kaliumbicarbonat | 20,26 |
| Citronensäure (wasserfrei) | 5,88 |
| Aspartam | 0,87 |
| Natriumsaccharin | 0,23 |
| trockenes Zitronenaroma | 1,59 |
| Zimtaroma | 0,61 |
| Natriumglycincarbonat | 48,77 |

13. Pharmazeutische Formulierung gemäß Anspruch 1, die ein freifließendes Pulver oder eine körnige Formulierung ist mit folgender Zusammensetzrung innerhalb von ±10%:
| Inhaltsstoff | Gew.-% |
|---|---|
| Amoxycillintrihydrat als freie Säure | 25,0 |
| Kaliumclavulanat als freie Säure | 2,08 |
| Natriumglycincarbonat | 41,39 |
| Kaliumbicarbonat | 20,86 |
| Citronensäure, wasserfrei | 5,33 |
| Aspartam | 1,25 |
| Natriumsaccharin | 0,33 |
| Siruparoma | 1,25 |
| Bananenaroma | 2,5 |

14. Pharmazeutische Formulierung gemäß Anspruch 1, in Form von Tabletten mit folgender Zusammensetzung innerhalb von ±10%:
| Inhaltsstoff | Gew.-% |
|---|---|
| Amoxycillintrihydrat als freie Säure | 10,43 |
| Kaliumclavulanat als freie Säure | 2,61 |
| Natriumglycincarbonat | 52,00 |
| Citronensäure, wasserfrei | 16,69 |
| Natriumbenzoat | 5,58 |
| Aspartam | 3,13 |
| Siruparoma | 3,13 |
| Bananenaroma | 6,26 |

15. Pharmazeutische Formulierung gemäß Anspruch 1 in Form von Tabletten mit folgender Zusammensetzung innerhalb von ±10%:
| Inhaltsstoff | Gew.-% |
|---|---|
| Amoxycillintrihydrat als freie Säure | 18,89 |
| Natriumglycincarbonat | 61,39 |
| Citronensäure, wasserfrei | 14,17 |
| Aspartam | 1,83 |
| Zitronensaftaroma | 2,69 |
| Zimtaroma | 1,03 |

16. Pharmazeutische Formulierung gemäß einem der vorstehenden Ansprüche, die eine Einheitsdosis Amoxycillin von 3000 bis 125 mg enthält.

17. Pharmazeutische Formulierung gemäß einem der vorstehenden Ansprüche zur Verwendung bei der Behandlung bakterieller Infektionen bei Mensch oder Tier.

18. Verfahren zur Herstellung einer Pharmazeutischen Formulierung zur oralen Verabreichung, umfassend das Vermischen eines Amoxycillinhydrats und eines Brausepaares, wobei das Paar eine saure Komponente und eine alkalische Komponente umfaßt und beim Kontakt mit Wasser Kohlendioxid freisetzt, und die alkalische Komponente im Überschuß zum stöchiometrischen Äquivalent der sauren Komponente in solcher Menge vorliegt, die ausreicht, um sowohl die saure Komponente zu neutralisieren als auch das Amoxycillin vollständig aufzulösen.

19. Verwendung eines Gemisches eines Amoxycillinhydrats und eines Brausepaares, wobei das Paar eine saure Komponente und eine alkalische Komponente umfaßt und beim Kontakt mit Wasser Kohlendioxid freisetzt, und die alkalische Komponente im Überschuß zum stöchiometrischen Äquivalent der sauren Komponente in solcher Menge vorliegt, die ausreicht, um sowohl die saure Komponente zu neutralisieren als auch das Amoxycillin vollständig aufzulösen, zur Herstellung eines oral verabreichbaren Arzneimittels zur Behandlung bakterieller Infektionen.

## Revendications

1. Formulation pharmaceutique pour administration orale comprenant un hydrate d'amoxycilline et un couple effervescent, le couple comprenant un composant acide et un composant alcalin générant du dioxyde de carbone au contact de l'eau, dans laquelle le composant alcalin du couple est présent en excès par rapport à l'équivalent stoechiométrique du composant acide, en quantité suffisante pour à la fois neutraliser le composant acide et pour solubiliser complètement l'amoxycilline.

2. Formulation pharmaceutique selon la revendication 1, dans laquelle l'hydrate d'amoxycilline est l'amoxycilline trihydrate et/ou est en conjonction avec un inhibiteur de β-lactamase.

3. Formulation pharmaceutique selon la revendication 2, dans laquelle l'inhibiteur de β-lactamase est présent, et est l'acide clavulanique ou un sel de celui-ci, dans un ratio de poids hydrate d'amoxycilline/inhibiteur compris entre 12/1 et 1/1.

4. Formulation pharmaceutique selon l'une quelconque des revendications précédentes, dans laquelle la proportion d'hydrate d'amoxycilline est de 10 à 30% en poids.

5. Formulation pharmaceutique selon l'une quelconque des revendications précédentes, dans laquelle le couple effervescent est choisi parmi l'acide citrique, l'acide tartrique ou l'acide malique ou des mélanges de ceux-ci en tant que composant acide, et le bicarbonate de sodium, le carbonate de glycine sodique ou le carbonate de sodium, ou les sels de potassium correspondants, ou des mélanges de ceux-ci, en tant que composant alcalin.

6. Formulation pharmaceutique selon l'une quelconque des revendications précédentes, qui, lorsqu'elle est passée en solution aqueuse, a un pH qui n'est pas inférieur à 8.

7. Formulation pharmaceutique selon l'une quelconque des revendications précédentes, qui contient 50 à 75 % en poids, de composant alcalin.

8. Formulation pharmaceutique selon l'une quelconque des revendications précédentes, dans laquelle le composant alcalin est un mélange 3 à 1,5/1, en poids, de carbonate de glycine sodique ou bicarbonate de sodium/bicarbonate de potassium.

9. Formulation pharmaceutique selon l'une quelconque des revendications précédentes, qui contient 2 à 25% en poids, de composant acide.

10. Formulation pharmaceutique selon l'une quelconque des revendications précédentes, comportant un ratio molaire carbonate de glycine sodique/amoxycilline/bicarbonate de potassium/acide citrique compris dans l'intervalle 4 à 10/1 à 3/5 à 10/1.

11. Formulation pharmaceutique selon la revendication 1, qui est une formulation sous forme de poudre coulant librement, ou sous forme de granulés, et ayant à ± 10% près, la composition :
| Ingrédient | % m/m |
|---|---|
| - Amoxycilline trihydrate (en acide libre) | 22,5 |
| - Bicarbonate de potassium | 21,04 |
| - Carbonate de glycine sodique | 46,7 |
| - Acide citrique | 6,01 |
| - Aspartame | 1,13 |
| - Saccharinate de sodium | 0,30 |
| - Arôme Jus de Citron | 1,65 |
| - Arôme Cannelle | 0,64 |

12. Formulation pharmaceutique selon la revendication 1, qui est une formulation sous forme de poudre coulant librement, ou sous forme de granulés, et ayant à ± 10% près, la composition :
| Ingrédient | % m/m |
|---|---|
| - Amoxycilline trihydrate (en équivalent amoxycilline acide libre) | 19,06 |
| - Clavulanate de potassium (en équivalent acide clavulanique) | 2,72 |
| - Bicarbonate de potassium | 20,26 |
| - Acide citrique (anhydre) | 5,88 |
| - Aspartame | 0,87 |
| - Saccharinate de sodium | 0,23 |
| - Arôme sec Citron | 1,59 |
| - Arôme Cannelle | 0,61 |
| - Carbonate de glycine sodique | 48,77 |

13. Formulation pharmaceutique selon la revendication 1, qui est une formulation sous forme de poudre coulant librement, ou sous forme de granulés, et ayant à ± 10 % près, la composition :
| Ingrédient | % m/m |
|---|---|
| - Amoxycilline trihydrate (en acide libre) | 25,0 |
| - Clavulanate de potassium (en acide libre) | 2,08 |
| - Carbonate de glycine sodique | 41,39 |
| - Bicarbonate de potassium | 20,86 |
| - Acide citrique anhydre | 5,33 |
| - Aspartame | 1,25 |
| - Saccharinate de sodium | 0,33 |
| - Arôme sirop Golden | 1,25 |
| - Arôme Banane | 2,5 |

14. Formulation pharmaceutique selon la revendication 1, qui est une formulation sous forme de comprimé ayant à ± 10 % près, la composition :
| Ingrédient | % m/m |
|---|---|
| - Amoxycilline trihydrate (en acide libre) | 10,43 |
| - Clavulanate de potassium (en acide libre) | 2,61 |
| - Carbonate de glycine sodique | 52,16 |
| - Acide citrique anhydre | 16,69 |
| - Benzoate de sodium | 5,58 |
| - Aspartame | 3,13 |
| - Arôme sirop Golden | 3,13 |
| - Arôme Banane | 6,26 |

15. Formulation pharmaceutique selon la revendication 1, qui est une formulation sous forme de comprimé ayant à ± 10 % près, la composition :
| Ingrédient | % m/m |
|---|---|
| - Amoxycilline trihydrate (en acide libre) | 18,89 |
| - Carbonate de glycine sodique | 61,39 |
| - Acide citrique anhydre | 14,17 |
| - Aspartame | 1,83 |
| - Arôme Jus de Citron | 2,69 |
| - Arôme Cannelle | 1,03 |

16. Formulation pharmaceutique selon l'une quelconque des revendications précédentes, contenant une dose unitaire d'amoxycilline comprise entre 3.000 et 125 mg, limites comprises.

17. Formulation pharmaceutique selon l'une quelconque des revendications précédentes, en vue du traitement d'infections bactériennes chez l'homme ou chez l'animal.

18. Procédé de préparation d'une formulation pharmaceutique pour administration orale, qui comprend le mélange d'un hydrate d'amoxycilline et d'un couple effervescent, le couple comprenant un composant acide et un composant alcalin qui génère du dioxyde de carbone au contact de l'eau, le composant alcalin du couple étant présent en excès par rapport à l'équivalent stoechiométrique du composant acide, en quantité suffisante pour à la fois neutraliser le composant acide et pour solubiliser complètement l'amoxycilline.

19. Utilisation d'un mélange d'un hydrate d'amoxycilline et d'un couple effervescent, le couple comprenant un composant acide et un composant alcalin qui génère du dioxyde de carbone au contact de l'eau, le composant alcalin du couple étant présent en excès par rapport a l'équivalent stoechiométrique du composant acide, en quantité suffisante pour à la fois neutraliser le composant acide et pour solubiliser complètement l'amoxycilline, dans la fabrication d'un médicament pour administration orale pour le traitement d'infections bactériennes.
